Europäisches Patentamt

European Patent Office

Office européen des brevets

(19)

(11) Publication number: **0 229 171 B1**

# EUROPEAN PATENT SPECIFICATION

(12)

(45) Date of publication of patent specification: **27.05.92**　(51) Int. Cl.⁵: **C12N 9/02**, C12N 1/14

(21) Application number: **86904671.4**

(22) Date of filing: **11.07.86**

(86) International application number:
**PCT/US86/01471**

(87) International publication number:
**WO 87/00550 (29.01.87 87/03)**

(54) NOVEL ENZYMES FOR DEGRADATION OF LIGNIN.

(30) Priority: **15.07.85 US 755242**
　　　　　　**28.03.86 US 845655**

(43) Date of publication of application:
**22.07.87 Bulletin 87/30**

(45) Publication of the grant of the patent:
**27.05.92 Bulletin 92/22**

(84) Designated Contracting States:
**AT BE CH DE FR GB IT LI LU NL SE**

(56) References cited:
**EP-A- 0 188 931**

**Proc. Natl. Acad. Sci. (USA) vol. 81, April 1984. M. Tien et al "Lignin degrading enzyme from Phanerochaete chrysosporium : purification, characterization and catalytic properties of a unique H2O2-requiring oxygenase" pages 2280-2284**

(73) Proprietor: **REPLIGEN CORPORATION
One Kendall Square Building 700
Cambridge, MA 02139(US)**

Proprietor: **THE UNITED STATES OF AMERICA as represented by THE SECRETARY OF AGRICULTURE
United States Department of Agriculture
Washington, DC 20250(US)**

(72) Inventor: **FARRELL, Roberta
177 Hobart Street
Danvers, MA 01923(US)**
Inventor: **KIRK, Thomas
7814 Ox Trail Way
Verona, WI 53593(US)**
Inventor: **TIEN, Ming
712 McKee Street
State College, PA 16803(US)**

EP 0 229 171 B1

Chemical abstracts, vol. 102, n 3, 21 January 1985 (Columbus, Ohio, US) M.H. Gold et al "Purification and characterization of an extracellular hydrogen peroxide requiring diarylpropane oxygenase from the white rot basidiomycete, phanerochaete chryso sporium", see page 302, abstract n 20116v, & Arch. Biochem. Biophys. 1984, 234(2), 353-62

Journal of biotechnology, vol. 2, n 6, 1985, Elsevier (NL) M. Leisola et al "Production and identification of extracellular oxidases of phanerochaete chrysosporium", pages 379-382

Journal of biotechnology, vol. 3 1985, Elsevier (NL) M.S.A. Leisola et al "Strategies for production of high ligninase activities by phanerochaete chrysosporium", pages 97-107

Chemical abstracts, vol. 101, n 7, 1 August 1094 (Columbus, Ohio, USA) M. Kuwahara et al "Separation and characterization of two extracellular hydeogen peroxide-dependent exidases from lignonolytic cultures of phanerochaete chrysosporium", see page 242, abstract n 50567q & FEBS Lett. 1984, 169(2), 247-50

Archives of Biochemistry and Biophysics, vol. 241, n 1, 15 August 1985, V. Renganathan et al "Multiple molecular forms of diaryl-propane oxygenase an H2O-requiring, lignin-degrading enzyme from phanerochaete chrysosporium", pages 304-314

Applied and environmental microbiology, vol. 50, n 5, November 1985, A. Jager et al "Production of ligninases and degradation of lignin in agitated submerged cultures of phanerochaete chrysosporium" pages 1274-1278

Enzyme microbiol. Technol. vol. 8, February 1986, T.K. Kirk et al "Lignin degradint activity of phanerochaete chrysosporium burds : comparison of cellulase negative and other strains" pages 75-80

Enzyme Microbiol. Technol., vol. 8, January 1986, T.K. Kirk et al "Production of multiple ligninases by phanerochaete chrysosporium : effect of selected growth conditions and use of a mutant strain" pages 27-32

Chemical abstracts, vol. 101, n 23, 3 December 1984 (Columbus, Ohio, US) M.H. Gold et al "Biochemical and genetic studies on lignin degradation by phanerochaete chrysosporium", see page 331, abstract n 207345r, & Recent Adv. Lignin Biodegrad. Res. (Proc. Int. Semin.) 2nd 1983, 219-32

Chemical abstracts, vol. 102, n 23, 10 June 1985 (Columbus, Ohio, US) S.C. Johnstrud et al "Cross-breeding of selected and mutated homo-karyotic strains of phanerochaete chrysosporium K-3 : new cellulase-deficient strains with increased ability to degrade lignin", see page 338, abstract n 200906m & Appl. Microbiol. Biothecnol. 1985, 21(5), 320-7

(74) Representative: **Holdcroft, James Gerald, Dr. et al**
Graham Watt & Co., Riverhead
Sevenoaks, Kent TN13 2BN(GB)

## Description

Background of the Invention

The invention disclosed herein is useful in several processes used by the pulp and paper industry. During pulping processes, cellulosic fibers must be liberated from their encasing lignin matrix so that they can associate with one another, yielding strength in the final product. This polymer separation can be accomplished by removal of lignin as in chemical pulps, or by maintaining the lignin as in high yield mechanical pulps. During the bleaching process, lignin is removed and the resulting pulp is brightened.

The secondary cell wall of wood, composed of cellulose fibrils, hemicellulose and lignin, imparts physical strength and rigidity to woody plants. The cellulose fibrils are densely packed and surround the cell in regular parallel arrays or in crisscross layers. These fibrils are held together by a matrix of hemicellulose and lignin.

Cellulose is the most abundant component of woody tissue, comprising 35-45% of the dry weight. Cellulose is an ordered linear polymer of glucose monomers coupled by $\beta$-1,4 bonds. The hemicelluloses are branched polymers composed of pentose (5-carbon) monomers, normally xylose and arabinose; and hexose (6-carbon) monomers, consisting of glucose, galactose, mannose and substituted uronic acid.

Lignin is an extremely complex polymer formed by the free radical polymerization of substituted cinnamyl alcohol precursors. Lignin constitutes 15-38% of dry wood weight.

Lignin is highly resistant to biological attack; not a surprising finding considering the complexity and stability of lignin structure. No organism has been demonstrated to grow on lignin as the sole carbon source. The complex lignin polymer, however, is completely degraded by pure cultures of various higher order fungi. For reviews see Higuchi (1982) Experientia 38: 159-166, and Janshekar, H. and Fiechter, A. (1983) "Advances in Biochemical Engineering/Bio- technology," A. Fiechter and T.W. Jeffries, Eds., Vol. 27, pp. 119-178, Springer, Berlin; Kirk, T.K. (1984) in "Biochemistry of Microbial Degradation," D.P. Gibson, Ed., pp. 399-437, Marcel Dekker, N.Y. The major degraders of "fully lignified" tissues (lignin > 20%) are the basiodiomycetes that cause the white-rot type of wood decay. The most extensive physiological investigations of lignin biodegrad- ation by white-rot fungi have been conducted with a single member of the family Corticeaceae, Phanerochaete chrysosporium Burds.

Although P. chrysosporium is capable of completely degrading lignin, purified lignin will not support its growth. Purified cellulose, however, is a growth nutrient for these fungi. Lignin degradation allows these fungi to expose the cellulose food source contained within the lignin matrix. Under defined laboratory conditions, fungal lignin degradation is not observed during the approximately first 3 days of culture. Subsequently, the culture becomes starved for carbon or nitrogen. Lignin degradation is first observed one or two days later and is maximal at 6 days. The induction of lignin degradation in response to carbon and nitrogen starvation indicated that fungal lignin metabolism is a secondary metabolic event (Keyser, P., Kirk, T.K. and Zeikus, J.G. (1978) J. Bacteriol. 135:790-797.)

Fungal lignin degradation is commercially impractical for several reasons. The rate of lignin degradation is unacceptably slow since ligninolytic activity must be induced by starvation. Furthermore, fungi metabolize cellulosic fibers as their primary food source, resulting in reduced pulp yield and an inferior pulp product.

With regard to the major C-C and C-O-C intersubunit linkages found in lignin, it is important to note that approximately 80% of intersubunit bonds involve linkages to the C$\alpha$ or C$\beta$ carbons.

Tien and Kirk have disclosed a preparation capable of oxidatively cleaving C$\alpha$ - C$\beta$ bonds in lignin model compounds (Tien, M. and Kirk, T.K. (1984) Proc. Natl. Acad. Sci. 81:2280-2284). This preparation displays on an SDS-polyacrylamide gel predominantly one protein with an apparent molecular weight of 42 kilodaltons and several minor bands.

Thus the preparation is a mixture of proteins without any means suggested for isolating the dominant protein from the minor bands. Subsequent to the publication of this paper, several scientific papers were published disclosing an inability to isolate the major protein from the mixture. These articles are as follows: Huynh, V-B and Crawford, R.L. (1985) FEMS Microbiology Letters 28:119-123; Leisola, M. et al. (1985) Lignin Biodegradation Workshop; and Gold, M.H. et al. (1985) Lignin Biodegradation Workshop.

These protein isolations have been done by either ion-exchange chromatography or size exclusion-ion exchange column chromatography. The fractions containing the indicated component have been analyzed by isoelectric focusing or SDS-polyacrylamide gel electrophoresis, and have shown multiple proteins. The scientists who performed this work are at the forefront of the lignin enzyme field, as evidenced by their participation in the Lignin Biodegradation Workshop held in Vancouver BC, in 1985. These failures suggest a seemingly insurmountable problem in resolving the prior art mixtures.

Isolation of the active component in the Tien and Kirk mixture is a highly desirable objective because, inter alia, the character of such mixtures has been found to substantially impede their practical use. Specifically, Tien and Kirk and similar mixtures have been found to be highly unstable at practical storage temperatures and at those temperatures suitable for effecting their practical activities. In particular, the useful activities of the Tien and Kirk mixture are substantially degraded in about 2 days time at room temperature and such mixtures have been found to contain destructive or protein degrading native proteases as indicated by the well-known azocoll test.

In addition, these is a clear need to isolate and identify other enzymes which can be used to catalyze the degradation and modification of lignin.

Brief Summary of the Invention

The invention disclosed herein has successfully solved the problem by producing a substantially pure enzyme preparation, herein designated rLDM™ 6, which is substantially free of destabilizing proteases. Advantageously, the rLDM™ 6 preparation of the present invention possesses desirable properties for practical usage which the Tien and Kirk preparation did not have.

Also, by working up the extracellular fluid from a culture of a novel strain of Phanerochaete chrysosporium in the manner hereinafter described, we have isolated not only rLDM™ 6, the apparent major protein in the Tien and Kirk mixture discussed above, but a series of other distinct lignin-degrading enzymes which are substantially free of destabilizing native proteases. These enzymes have stability suitable for practical use, are immediately active and require no metabolic induction.

The enzymes provided by the invention not only degrade lignin and effect various reactions as indicated on lignin model compounds but also will not attack cellulose or hemicellulose, and are further indicated, as of particular significance, as useful in the selective degradation and modification of lignin in wood pulp, and hence, are indicated for use in operations in the pulp and paper industry in which such a result is sought. The various properties of the enzyme preparation of the invention including their substantial purity which frees the enzymes from activity degrading native proteins is of distinct advantage of such usage.

Hence, the subject invention concerns novel lignin-degrading enzymes which are called rLDM™ 1, rLDM™ 2, rLDM™ 3, rLDM™ 4, rLDM™ 5 and rLDM™ 6, each substantially free of native protein which naturally degrades these enzymes.

These novel compounds, which are defined in claims 1 to 5, advantageously, possess the properties of (1) reducing the amount of lignin in kraft pulp, (2) enhancing the strength properties of thermomechanical pulp (TMP) and (3) decolorizing kraft lignin. The rLDM™ of the subject invention, as defined in claims 1 to 5 are characterized herein by the critical property of being able to catalyze the oxidation of veratryl alcohol to veratrylaldehyde, and the following physical parameters:

(1) molecular weight as determined by SOS-PAGE;
(2) amino acid compositions;
(3) heme content;
(4) homology of antibody reactivity;
(5) specificity of activity against lignin model substrates; and
(6) elution from a FPLC column at specified sodium acetate molarities.

The lignin-degrading enzymes of the invention referred to as rLDM™, herein, have previously been referred to as Pulpases™.

Detailed Description of the Invention

The isolation of the novel rLDM™ of the subject invention was facilitated by use of a novel stable mutant strain of Phanerochaete chrysosporium, which elaborates high amounts of ligninolytic enzymes into the fermentation medium. The novel mutant strain, designated SC26, has been deposited in the permanent collection of a public culture repository, to be maintained for at least 30 years. The culture repository is the Northern Regional Research Laboratory, U.S. Department of Agriculture, Peoria, Illinois 61604, USA. The accession number is NRRL 15978, and the deposit date is July 3, 1985. This deposited culture is available to the public as required by patent laws in countries wherein counterparts of the subject application, or its progeny, are filed. However, it should be understood that the availability of a deposit does not constitute a license to practice the subject invention in derogation of patent rights granted by governmental action.

Novel mutant SC26 was obtained by UV mutagenesis of the wild type Phanerochaete chrysosporium, ATCC 24725.

Novel mutant SC26 was grown on a nitrogen-limited trace element medium supplemented with glucose

4

and buffered at pH 4.5

Ligninase activity in the fermentation medium was measured periodically by standard means determining the rate of oxidation of veratryl alcohol to veratrylaldehyde.

Isolation and purification of the novel rLDM™ of the subject invention from the extracellular fluid in the fermentation was accomplished by ultrafiltration and FPLC using an anion exchange column.

Following are examples which illustrate the novel enzymes and procedures, including the best mode, for practicing the invention. These examples should not be construed as limiting. All percentages are by weight and all solvent mixture proportions are by volume unless otherwise noted.

Example 1 - Growth of Mutant SC26 (NRRL 15978) to Produce Fermentation Medium Containing Novel Ligninases

Inoculum was prepared by homogenizing 50 ml of 1.5-day cultures of mutant SC26 grown in 1 liter flasks containing the following medium, designated nitrogen-limited BIII/glucose medium:

The BIII medium contains $1.08 \times 10^{-3}$ M ammonium tartrate, $1.47 \times 10^{-2}$ M $KH_2PO_4$, $2.03 \times 10^{-3}$ M $MgSO_4.7H_2O$, $6.8 \times 10^{-4}$ M $CaCl_2.2H_2O$, $2.96 \times 10^{-6}$ M thiamine.HCl and 10 ml.$L^{-1}$ of a trace element solution. The trace element solution contain $7.8 \times 10^{-3}$ M nitriloacetic acid, $1.2 \times 10^{-2}$ M $MgSO_4.7H_2O$, $1.7 \times 10^{-2}$ M NaCl, $3.59 \times 10^{-4}$ M $FeSO_4.7H_2O$, $7.75 \times 10^{-4}$ M $CoCl_2$, $9.0 \times 10^{-4}$ M $CaCl_2$, $3.48 \times 10^{-4}$ M $ZnSO_4.7H_2O$, $4 \times 10^{-5}$ M $CuSO_4.5H_2O$, $2.1 \times 10^{-5}$ M $AlK(SO_4)_2.12H_2O$, $1.6 \times 10^{-4}$ M $H_3BO_3$, $4.1 \times 10^{-5}$ M $NaMoO_4.2H_2O$ and $2.9 \times 10^{-3}$ M $MnSO_4.H_2O$.

The medium was supplemented with 10% (by wt/liter) of glucose.

The medium was buffered with 10 mM transaconitic acid, pH 4.5.

Flasks (125 ml, containing 10 ml sterile medium having the above-described medium) were each inoculated with 0.5 ml of the above homogenate and kept stationary at 39°C. The flasks were flushed on days 0, 3 and 6 with water-saturated $O_2$. Alternatively, a rotating biological contractor (RBC) was used to grow the fungus. 2.5 liters of the above-described medium was inoculated with 100 ml of the above homogenate and grown at 39°C with the RBC rotating at 1 rpm with continuous oxygenation.

Ligninase activity was measured periodically by determining the rate of oxidation of veratryl alcohol to veratrylaldehyde. Reaction mixtures contained 275 μl of extracellular fluid (from flasks or the RBC), 2 mM veratryl alcohol, 0,4 mM $H_2O_2$ and 0.1 mM sodium tartrate, pH 2.5 in a final volume of 0.5 ml. The reactions were started by $H_2O_2$ addition immediately after buffer was added and were monitored at 310 nm. Protein was determined according to Bradford (Bradford, M.M. (1976) Anal. Biochem. 72:248-254) using bovine serum albumin (Sigma Chemical, St. Louis, MO) as standard.

Example 2 - Isolation and Purification of the Novel rLDM™

The extracellular growth media from cultures grown in flasks, as described above, was harvested by centrifugation at 5000 x G, 10 min, 4°C. Extracellular growth media was then concentrated by ultrafiltration through a 10K filter. The resulting concentrate is called the Ligninolytic Mixture™. The ligninolytic Mixture™ can contain one or more of rLDM™s or other ligninolytic enzymes in varying proportions. The rLDM™ contained in this Ligninolytic Mixture™ were separated by fast protein liquid chromatography (FPLC) using a Pharmacia Mono Q column (Pharmacia, Piscataway, NJ) and a gradient of sodium acetate buffer, pH 6, from 10 mM to 1 M. rLDM™ 1, 2, 3, 4, 5 and 6 elute from the column in a typical preparation at the following sodium acetate molarities, respectively: 0.16, 0.18, 0.34, 0.40, 0.58 and 0.43 M to give essentially pure rLDM™ 1-6. Each rLDM™ is substantially free of other rLDM™ and native proteins including substantial freedom from undesirable native destructive proteases. There are indications of these proteases in crude mixtures which are difficult to separate (each substantially pure rLDM™ gives a negative result in the Azocoll test).

Characterization of the Novel rLDM™

The rLDM™ have been characterized by the following criteria:
(1) ability to catalyze the oxidation of veratryl alcohol to veratrylaldehyde;
(2) molecular weight as determined by SDS-PAGE;
(3) amino acid composition;
(4) heme content:
(5) homology by antibody reactivity;
(6) specificity of activity against lignin model substrates; and

(7) elution from an FPLC column at specified sodium acetate molarities.

All of the rLDM™ catalyze the oxidation of veratryl alcohol to veratrylaldehyde, as monitored spectrophotometrically at 310 nm. A unit of activity is defined as the production of 1 micromole of veratrylaldehyde in the rLDM™ catalyzed reaction. The specific activities of typical preparations at about 24°C are as follows:

| rLDM™ | | | | | | |
|---|---|---|---|---|---|---|
| | 1 | 2 | 3 | 4 | 5 | 6 |
| Specific Activity Units/MG Minute | 2.6 | 17.1 | 5.1 | 9.7 | 9.4 | 12.4 |
| Molecular Weight kD | 38 | 38 | 42 | 42 | 43 | 42 |

Amino acid composition - Amino acid composition was determined by a modification of the procedure of Jones et al. (Jones, B.N., Paabo, S. and Stein, S. (1981) J. Liquid Chromatography 4:565-586). The ratio of amino acids is approximately due to the limitation of technique and quantity of protein used in the determination. See Table 1

Heme and carbohydrate content - rLDM™ 1, 2, 3, 4, 5 and 6 each contain a single protoheme IX moiety. All are glycosylated according to periodic acid staining (PAS) and binding to Con A-Sepharose (Sigma).

Immunoblot Procedure

This procedure was used to further characterize the rLDM™. It is a standard procedure which is disclosed in Towbin et al. (Towbin, H., Staehelin, T. and Gordon, J. (1979) Proc. Natl. Acad. Sci. USA 76:4350). The procedure involves separating the proteins by electrophoresis in a gel, transfer of the proteins to a solid matrix and reacting with (1) a primary probe, rabbit anti-rLDM™ antibody and (2) a secondary probe, goat anti-rabbit antibody coupled to horseradish peroxidase.

rLDM™ 1, 3, 4, 5 and 6 react to polyclonal antibodies made to rLDM™ 2 and 6, using the above immunoblot procedure. rLDM™ 2, in the same procedure, reacts to polyclonal antibodies made to rLDM™ 6.

All the rLDM™ disclosed herein have the following unique activities on lignin model substrates, i.e., veratryl alcohol, 1-(3',4'-dimethoxy phenyl)glycerol-$\beta$-guaiacyl ether, phenol, methoxylated benzenes such as 1,4-dimethoxybenzene:

(1) oxidative cleavage of C$\alpha$-C$\beta$;

(2) hydroxylation of benzylic methylene groups;

(3) oxidation of benzyl alcohols to aldehydes;

(4) phenol oxidation; and

(5) oxidative cleavage of methoxyl groups.

"Lignin model substrates" are chemicals which resemble parts of lignin. The reaction products of the model compounds with rLDM™s can have practical utility particularly to but not limited to food, pharmaceutical and chemical industries as chemical feedstocks. The above activities are characteristic of the rLDM™ disclosed herein.

Example 3 - Bleaching of Kraft Pulp with rLDM™

rLDM™ 1-6, alone, or mixtures thereof, are added to kraft pulp having a characteristic brown color at 3% consistency in 10mM transaconitic acid, pH 4.5, 400 $\mu$M $H_2O_2$ and 100 $\mu$M $MnSO_4$. The pulp slurry is flushed with $O_2$ and incubated with slow shaking at 39°C for 12 hrs., after which the kraft pulp solution is decanted, and a 1M NaOH solution is added to the pulp and incubated for 60 min. at 65°C. This is then decanted and the kraft pulp is washed in water. The resulting kraft pulp no longer has a dark brown color, but instead has a desired lighter color.

The use of $MnSO_4$ is optional.

Example 4 - Treatment of Thermomechanical Pulp (TMP) with rLDM™

rLDM™ 1-6, alone, or mixtures thereof, are added to 10 gm of TMP (dry weight) at 3% consistency in 10 mM trans-aconitic acid, pH 4.5, 400 $\mu$M $H_2O_2$ and 100 $\mu$M $MnSO_4$. The pulp slurry is flushed with $O_2$ and incubated with slow shaking at 39°C for 12 hr., after which time the TMP is washed with water. The tensile, tear and burst indices as well as breaking length of the pulp measured and found to be of enhanced strength versus an untreated sample. The brightness reversion of the treated sample is less than the untreated sample; therefore, brightness stability is increased with the rLDM™ treatment.

The use of $MnSO_4$ is optional.

The rLDM™ of the subject invention can be used in a purified form, wherein each rLDM™ is substantially free of other rLDM™ and native proteins, and in mixtures thereof. It is well within the skill of a person skilled in the art to adjust amounts of rLDM™ used in accordance with the purity of the rLDM™ preparation. The rLDM™s may be combined with various diluents, adjuvants and other chemicals including proteins which are non-deleterious to the rLDM™s and their use, for various purposes such as providing marketable forms and enhancing their use.

"Native proteins" as used herein refers to other proteins present in the extracellular fermentation medium, as described above.

Example 5 - Treatment of Softwood Kraft Pulp with rLDM™

One part of softwood kraft pulp is treated with about $10 \times 10^{-6}$ to about $20 \times 10^{-6}$ parts of a rLDM™ in about 40 mM trans-aconitic acid, pH 4.5, at about 39°C for about 1 to about 16 hrs. The pulp is then washed in about 1 M NaOH at about 65°C for about 1 hour, and rinsed in water. This treatment of the softwood kraft pulp results in the removal of about 1/3 of the lignin as evidenced by the reduction of kappa number from about 18 to about 13.

## Claims
### Claims for the following Contracting States : BE, CH, DE, FR, GB, IT, LI, LU, NL, SE

1. The ligninolytic enzyme (A) which is substantially free of proteases and has the following characteristics:

   (a) 1 mg catalyzes in one minute at about 24°C the oxidation of about 12.4 micromole of veratryl alcohol to veratryl aldehyde;
   (b) has a molecular weight of about 42 kilo-daltons;
   (c) contains a single protoheme IX moiety;
   (d) is glycosylated;
   (e) contains the following molar proportions of amino acids based on one mole of lysine:

   | asp/asn | 3.0 | thr | 4.9 | val 4.2 |
   |---------|-----|-----|------|---------|
   | glu/gln | 8.0 | arg | 1.3 | phe 3.2 |
   | ser | 6.8 | ala | 6.7 | ile 2.4 |
   | his | 3.2 | tyr | 0.2 | leu 3.3 |
   | gly | 8.3 | met | 0.14 | lys 1.0 |

   (f) elutes from an FPLC column at a sodium acetate molarity of about 0.43; and
   (g) has the following activities on lignin model substrates;
      (1) oxidative cleavage of $C\alpha$-$C\beta$;
      (2) hydroxylation of benzylic methylene groups;
      (3) oxidation of benzyl alcohols to aldehydes;
      (4) phenol oxidation; and
      (5) oxidative cleavage of methoxyl groups; or
   the ligninolytic enzyme (B) which is substantially free of proteases, and has the following characteristics:

   (a) 1 mg catalyzes in one minute at about 24°C the oxidation of about 17.1 micromole of veratryl alcohol to veratryl aldehyde;
   (b) has a molecular weight of about 38 kilo-daltons;
   (c) contains a single protoheme IX moiety;
   (d) is glycosylated;
   (e) contains the following molar proportions of amino acids based on one mole of lysine:

| asp/asn | 2.0 | thr | 3.5 | | val | 2.6 |
|---------|-----|-----|-----|---|-----|-----|
| glu/gln | 7.7 | arg | 1.2 | | phe | 3.0 |
| ser | 4.1 | ala | 7.9 | | ile | 2.2 |
| his | 3.2 | tyr | not determined | | leu | 2.6 |
| gly | 5.7 | met | not determined | | lys | 1.0 |

(f) elutes from an FPLC column at a sodium acetate molarity of about 0.18; and

(g) has the following activities on lignin model substrates;

    (1) oxidative cleavage of $C\alpha$-$C\beta$;

    (2) hydroxylation of benzylic methylene groups;

    (3) oxidation of benzyl alcohols to aldehydes;

    (4) phenol oxidation; and

    (5) oxidative cleavage of methoxyl groups;

said ligninolytic enzyme (A) having the characteristic of reacting in an immunoblot procedure to polyclonal antibodies made to the ligninolytic enzymes (A) and (B); and said ligninolytic enzyme (B) having the characteristic of reacting in an immunoblot procedure to polyclonal antibodies made to the ligninolytic enzyme (A).

2. The ligninolytic enzyme, which is substantially free of proteases, and has the following characteristics:

(a) 1 mg catalyzes in one minute at about 24°C the oxidation of about 2.6 micromole of veratryl alcohol to veratryl aldehyde;

(b) has a molecular weight of about 38 kilo-daltons;

(c) contains a single protoheme IX moiety;

(d) is glycosylated;

(e) contains the following molar proportions of amino acids based on one mole of isoleucine:

| asp/asn | 1.4 | thr | 2.2 | | val | 1.6 |
|---------|-----|-----|-----|---|-----|-----|
| glu/gln | 6.0 | arg | 1.1 | | phe | 1.1 |
| ser | 4.3 | ala | 7.3 | | ile | 1.0 |
| his | 4.4 | tyr | 0.2 | | leu | 1.5 |
| gly | 6.5 | met | not determined | | lys | 0.5 |

(f) reacts in an immunoblot procedure to polyclonal antibodies made to ligninolytic enzymes (A) and (B) of claim 1;

(g) elutes from an FPLC column at a sodium acetate molarity of about 0.16; and

(h) has the following activities on lignin model substrates;

    (1) oxidative cleavage of $C\alpha$-$C\beta$;

    (2) hydroxylation of benzylic methylene groups;

    (3) oxidation of benzyl alcohols to aldehydes;

    (4) phenol oxidation; and

    (5) oxidative cleavage of methoxyl groups.

3. The ligninolytic enzyme, which is substantially free of proteases, and has the following characteristics:

(a) 1 mg catalyzes in one minute at about 24°C the oxidation of about 5.1 micromole of veratryl alcohol to veratryl aldehyde;

(b) has a molecular weight of about 42 kilo-daltons;

(c) contains a single protoheme IX moiety;

(d) is glycosylated;

(e) contains the following molar proportions of amino acids based on one mole of tyrosine:

| asp/asn | 5.4 | thr | not determined | val | 7.4 |
|---------|------|-----|----------------|-----|-----|
| glu/gln | 16.8 | arg | 2.9 | phe | 7.0 |
| ser | 14.0 | ala | 14.4 | ile | 4.1 |
| his | 7.3 | tyr | 1.0 | leu | 6.5 |
| gly | 24.0 | met | 1.2 | lys | 2.5 |

(f) reacts in an immunoblot procedure to polyclonal antibodies made to ligninolytic enzymes (A) and (B) of claim 1;

(g) elutes from an FPLC column at a sodium acetate molarity of about 0.34; and

(h) has the following activities on lignin model substrates;

    (1) oxidative cleavage of $C\alpha$-$C\beta$;

    (2) hydroxylation of benzylic methylene groups;

    (3) oxidation of benzyl alcohols to aldehydes;

    (4) phenol oxidation; and

    (5) oxidative cleavage of methoxyl groups.

4. The ligninolytic enzyme, which is substantially free of proteases, and has the following characteristics:

    (a) 1 mg catalyzes in one minute at about 24°C the oxidation of about 9.7 micromole of veratryl alcohol to veratryl aldehyde;

    (b) has a molecular weight of about 42 kilo-daltons;

    (c) contains a single protoheme IX moiety;

    (d) is glycosylated;

    (e) reacts in an immunoblot procedure to polyclonal antibodies made to ligninolytic enzymes (A) and (B) of claim 1;

    (f) elutes from an FPLC column at a sodium acetate molarity of about 0.40; and

    (g) has the following activities on lignin model substrates;

        (1) oxidative cleavage of $C\alpha$-$C\beta$;

        (2) hydroxylation of benzylic methylene groups;

        (3) oxidation of benzyl alcohols to aldehydes;

        (4) phenol oxidation; and

        (5) oxidative cleavage of methoxyl groups.

5. The ligninolytic enzyme, which is substantially free of proteases, and has the following characteristics:

    (a) 1 mg catalyzes in one minute at about 24°C the oxidation of about 9.4 micromole of veratryl alcohol to veratryl aldehyde;

    (b) has a molecular weight of about 43 kilo-daltons;

    (c) contains a single protoheme IX moiety;

    (d) is glycosylated;

    (e) contains the following proportions of amino acids based on one mole of tyrosine:

| asp/asn | 5.0 | thr | not determined | val | 6.5 |
|---------|------|-----|----------------|-----|-----|
| glu/gln | 19.9 | arg | 4.8 | phe | 3.3 |
| ser | 22.3 | ala | 13.8 | ile | 3.6 |
| his | 15.9 | tyr | 1.0 | leu | 6.0 |
| gly | 44.7 | met | not determined | lys | 2.3 |

    (f) reacts in an immunoblot procedure to polyclonal antibodies made to ligninolytic enzymes (A) and (B) of claim 1;

    (g) elutes from an FPLC column at a sodium acetate molarity of about 0.58; and

    (h) has the following activities on lignin model substrates;

(1) oxidative cleavage of Cα-Cβ;
(2) hydroxylation of benzylic methylene groups;
(3) oxidation of benzyl alcohols to aldehydes;
(4) phenol oxidation; and
(5) oxidative cleavage of methoxyl groups.

6. A biologically pure mutant culture of Phanerochaete chrysosporium, designated mutant SC26, and having the culture deposit number NRRL 15978, which mutant, upon being grown in a suitable medium, elaborates ligninolytic enzymes according to the preceding claims.

7. A process of treating a material susceptible to breakdown by ligninolytic enzymes characterized in that said material is treated with a ligninolytic enzyme according to any one of claims 1 to 5.

8. A process according to claim 7, wherein said material comprises lignin.

9. A process according to claim 7, wherein said material comprises any of the lignin model substrates: veratryl alcohol; 1-(3', 4'-dimethoxyphenyl) glycerol-β-guaiacyl ether; phenol; and a methoxylated benzene, e.g. 1,4-dimethoxybenzene.

10. A process according to claim 8, wherein said material is kraft pulp or thermomechanical pulp.

**Claims for the following Contracting State : AT**

1. A process for the preparation of the ligninolytic enzyme (A) which is substantially free of proteases and has the following characteristics:
   (a) 1 mg catalyzes in one minute at about 24°C the oxidation of about 12.4 micromole of veratryl alcohol to veratryl aldehyde;
   (b) has a molecular weight of about 42 kilo-daltons;
   (c) contains a single protoheme IX moiety;
   (d) is glycosylated;
   (e) contains the following molar proportions of amino acids based on one mole of lysine:

| asp/asn | 3.0 | thr | 4.9 | val | 4.2 |
| glu/gln | 8.0 | arg | 1.3 | phe | 3.2 |
| ser | 6.8 | ala | 6.7 | ile | 2.4 |
| his | 3.2 | tyr | 0.2 | leu | 3.3 |
| gly | 8.3 | met | 0.14 | lys | 1.0 |

   (f) elutes from an FPLC column at a sodium acetate molarity of about 0.43; and
   (g) has the following activities on lignin model substrates;
      (1) oxidative cleavage of Cα-Cβ;
      (2) hydroxylation of benzylic methylene groups;
      (3) oxidation of benzyl alcohols to aldehydes;
      (4) phenol oxidation; and
      (5) oxidative cleavage of methoxyl groups; or
the ligninolytic enzyme (B) which is substantially free of proteases, and has the following characteristics:
   (a) 1 mg catalyzes in one minute at about 24°C the oxidation of about 17.1 micromole of veratryl alcohol to veratryl aldehyde;
   (b) has a molecular weight of about 38 kilo-daltons;
   (c) contains a single protoheme IX moiety;
   (d) is glycosylated;
   (e) contains the following molar proportions of amino acids based on one mole of lysine:

| asp/asn | 2.0 | thr | 3.5 | | val | 2.6 |
|---------|-----|-----|-----|---|-----|-----|
| glu/gln | 7.7 | arg | 1.2 | | phe | 3.0 |
| ser | 4.1 | ala | 7.9 | | ile | 2.2 |
| his | 3.2 | tyr | not determined | | leu | 2.6 |
| gly | 5.7 | met | not determined | | lys | 1.0 |

(f) elutes from an FPLC column at a sodium acetate molarity of about 0.18; and

(g) has the following activities on lignin model substrates;

    (1) oxidative cleavage of C$\alpha$-C$\beta$;

    (2) hydroxylation of benzylic methylene groups;

    (3) oxidation of benzyl alcohols to aldehydes;

    (4) phenol oxidation; and

    (5) oxidative cleavage of methoxyl groups;

said ligninolytic enzyme (A) having the characteristic of reacting in an immunoblot procedure to polyclonal antibodies made to the ligninolytic enzymes (A) and (B); and said ligninolytic enzyme (B) having the characteristic of reacting in an immunoblot procedure to polyclonal antibodies made to the ligninolytic enzyme (A) which process comprises working up the extracellular fluid from a culture of Phanerochaete chrysosporium strain SC26 (NRRL 15978), and separating enzyme A, or enzyme B by means of anion exchange chromatography.

2. A process for the preparation of the ligninolytic enzyme, which is substantially free of proteases, and has the following characteristics:

    (a) 1 mg catalyzes in one minute at about 24°C the oxidation of about 2.6 micromole of veratryl alcohol to veratryl aldehyde;

    (b) has a molecular weight of about 38 kilo-daltons;

    (c) contains a single protoheme IX moiety;

    (d) is glycosylated;

    (e) contains the following molar proportions of amino acids based on one mole of isoleucine:

| asp/asn | 1.4 | thr | 2.2 | | val 1.6 |
|---------|-----|-----|-----|---|---------|
| glu/gln | 6.0 | arg | 1.1 | | phe 1.1 |
| ser | 4.3 | ala | 7.3 | | ile 1.0 |
| his | 4.4 | tyr | 0.2 | | leu 1.5 |
| gly | 6.5 | met | not determined | | lys 0.5 |

    (f) reacts in an immunoblot procedure to polyclonal antibodies made to ligninolytic enzymes (A) and (B) of claim 1;

    (g) elutes from an FPLC column at a sodium acetate molarity of about 0.16; and

    (h) has the following activities on lignin model substrates;

        (1) oxidative cleavage of C$\alpha$-C$\beta$;

        (2) hydroxylation of benzylic methylene groups;

        (3) oxidation of benzyl alcohols to aldehydes;

        (4) phenol oxidation; and

        (5) oxidative cleavage of methoxyl groups,

which process comprises working up the extracellular fluid from a culture of Phanerochaete chrysosporium strain SC26 (NRRL 15978), and separating said enzyme by means of anion exchange chromatography.

3. A process for the preparation of the ligninolytic enzyme, which is substantially free of proteases, and has the following characteristics:

    (a) 1 mg catalyzes in one minute at about 24°C the oxidation of about 5.1 micromole of veratryl alcohol to veratryl aldehyde;

    (b) has a molecular weight of about 42 kilo-daltons;

    (c) contains a single protoheme IX moiety;

    (d) is glycosylated;

    (e) contains the following molar proportions of amino acids based on one mole of tyrosine:

| | | | | | |
|---|---|---|---|---|---|
| asp/asn | 5.4 | thr | not determined | val | 7.4 |
| glu/gln | 16.8 | arg | 2.9 | phe | 7.0 |
| ser | 14.0 | ala | 14.4 | ile | 4.1 |
| his | 7.3 | tyr | 1.0 | leu | 6.5 |
| gly | 24.0 | met | 1.2 | lys | 2.5 |

(f) reacts in an immunoblot procedure to polyclonal antibodies made to ligninolytic enzymes (A) and (B) of claim 1;

(g) elutes from an FPLC column at a sodium acetate molarity of about 0.34; and

(h) has the following activities on lignin model substrates;

(1) oxidative cleavage of C$\alpha$-C$\beta$;

(2) hydroxylation of benzylic methylene groups;

(3) oxidation of benzyl alcohols to aldehydes;

(4) phenol oxidation; and

(5) oxidative cleavage of methoxyl groups

which process comprises working up the extracellular fluid from a culture of Phanerochaete chrysosporium strain SC26 (NRRL 15978), and separating said enzyme by means of anion exchange chromatography.

**4.** A process for the preparation of the ligninolytic enzyme, which is substantially free of proteases, and has the following characteristics:

(a) 1 mg catalyzes in one minute at about 24°C the oxidation of about 9.7 micromole of veratryl alcohol to veratryl aldehyde;

(b) has a molecular weight of about 42 kilo-daltons;

(c) contains a single protoheme IX moiety;

(d) is glycosylated;

(e) reacts in an immunoblot procedure to polyclonal antibodies made to ligninolytic enzymes (A) and (B) of claim 1;

(f) elutes from an FPLC column at a sodium acetate molarity of about 0.40; and

(g) has the following activities on lignin model substrates:

(1) oxidative cleavage of C$\alpha$-C$\beta$;

(2) hydroxylation of benzylic methylene groups;

(3) oxidation of benzyl alcohols to aldehydes;

(4) phenol oxidation; and

(5) oxidative cleavage of methoxyl groups

which process comprises working up the extracellular fluid from a culture of Phanerochaete chrysosporium strain SC26 (NRRL 15978), and separating said enzyme by means of anion exchange chromatography.

**5.** A process for the preparation of the ligninolytic enzyme, which is substantially free of proteases, and has the following characteristics:

(a) 1 mg catalyzes in one minute at about 24°C the oxidation of about 9.4 micromole of veratryl alcohol to veratryl aldehyde;

(b) has a molecular weight of about 43 kilo-daltons;

(c) contains a single protoheme IX moiety;

(d) is glycosylated;

(e) contains the following proportions of amino acids based on one mole of tyrosine:

| asp/asn | 5.0 | thr | not determined | val | 6.5 |
|---------|------|-----|----------------|-----|-----|
| glu/gln | 19.9 | arg | 4.8 | phe | 3.3 |
| ser | 22.3 | ala | 13.8 | ile | 3.6 |
| his | 15.9 | tyr | 1.0 | leu | 6.0 |
| gly | 44.7 | met | not determined | lys | 2.3 |

(f) reacts in an immunoblot procedure to polyclonal antibodies made to ligninolytic enzymes (A) and (B) of claim 1;

(g) elutes from an FPLC column at a sodium acetate molarity of about 0.58; and

(h) has the following activities on lignin model substrates:

(1) oxidative cleavage of C$\alpha$-C$\beta$;

(2) hydroxylation of benzylic methylene groups;

(3) oxidation of benzyl alcohols to aldehydes;

(4) phenol oxidation; and

(5) oxidative cleavage of methoxyl groups,

which process comprises working up the extracellular fluid from a culture of Phanerochaete chrysosporium strain SC26 (NRRL 15978), and separating said enzyme by means of anion exchange chromatography.

6. A process for preparing a biologically pure mutant culture of Phanerochaete chrysosporium, designated mutant SC26, and having the culture deposit number NRRL 15978, which process comprises inoculating a suitable medium with said mutant culture and growing the culture under conditions suitable for allowing said culture to elaborate ligninolytic enzymes according to the preceding claims.

7. A process of treating a material susceptible to breakdown by ligninolytic enzymes characterized in that said material is treated with a ligninolytic enzyme according to any one of claims 1 to 5.

8. A process according to claim 7, wherein said material comprises lignin.

9. A process according to claim 7, wherein said material comprises any of the lignin model substrates:

veratryl alcohol; 1-(3', 4'-dimethoxyphenyl) glycerol-$\beta$-guaiacyl ether; phenol; and a methoxylated benzene, e.g. 1,4-dimethoxybenzene.

10. A process according to claim 8, wherein said material is kraft pulp or thermomechanical pulp.

**Revendications**
**Revendications pour les Etats contractants suivants : BE, CH, DE, FR, GB, IT, LI, LU, NL, SE**

1. L'enzyme ligninolytique (A) qui est substantiellement exempte de protéases et a les caractéristiques suivantes:

(a) 1 mg catalyse en une minute à environ 24°C l'oxydation d'environ 12,4 micromoles d'alcool vératrylique en aldéhyde vératrique;

(b) a un poids moléculaire d'environ 42 kilo-daltons;

(c) contient un seul reste protohème IX;

(d) est glycosylée;

(e) contient les proportions molaires suivantes en amino-acides basées sur une mole de lysine :

| asp/asn | 3,0 | thr | 4,9 | val | 4,2 |
|---------|-----|-----|------|-----|-----|
| glu/gln | 8,0 | arg | 1,3 | phe | 3,2 |
| ser | 6,8 | ala | 6,7 | ile | 2,4 |
| his | 3,2 | tyr | 0,2 | leu | 3,3 |
| gly | 8,3 | met | 0,14 | lys | 1,0 |

(f) est éluée sur colonne FPLC à une molarité d'acétate de sodium d'environ 0,43; et

(g) a les activités suivantes sur des substrats modèles de la lignine;

    (1) clivage oxydant de $C_\alpha$-$C_\beta$;

    (2) hydroxylation des groupes méthylène benzyliques;

    (3) oxydation des alcools benzyliques en aldéhydes;

    (4) oxydation des phénols; et

    (5) clivage oxydant des groupes méthoxy, ou

l'enzyme ligninolytique (B) qui est substantiellement exempte de protéases et a les caractéristiques suivantes :

(a) 1 mg catalyse en une minute à environ 24°C l'oxydation d'environ 17,1 micromoles d'alcool vératrylique en aldéhyde vératrique;

(b) a un poids moléculaire d'environ 38 kilo-daltons;

(c) contient un seul reste protohème IX;

(d) est glycosylée;

(e) contient les proportions molaires suivantes en amino-acides basées sur une mole de lysine:

| asp/asn | 2,0 | thr | 3,5 | val | 2,6 |
|---------|-----|-----|-----|-----|-----|
| glu/gln | 7,7 | arg | 1,2 | phe | 3,0 |
| ser | 4,1 | ala | 7,9 | ile | 2,2 |
| his | 3,2 | tyr | non déterminé | leu | 2,6 |
| gly | 5,7 | met | non déterminé | lys | 1,0 |

(f) est éluée sur colonne FPLC à une molarité d'acétate de sodium d'environ 0,18; et

(g) a les activités suivantes sur des substrats modèles de la lignine ;

    (1) clivage oxydant de $C_\alpha$-$C_\beta$;

    (2) hydroxylation des groupes méthylène benzyliques;

    (3) oxydation des alcools benzyliques en aldéhydes;

    (4) oxydation des phénols; et

    (5) clivage oxydant des groupes méthoxy;

ladite enzyme ligninolytique (A) ayant la caractéristique de réagir dans un procédé immunoblot avec des anticorps polyclonaux dirigés contre les enzymes ligninolytiques (A) et (B); et ladite enzyme ligninolytique (B) ayant la caractéristique de réagir dans un procédé immunoblot avec des anticorps polyclonaux dirigés contre l'enzyme ligninolytique (A).

2. L'enzyme ligninolytique qui est substantiellement exempte de protéases et a les caractéristiques suivantes :

(a) 1 mg catalyse en une minute à environ 24°C l'oxydation d'environ 2,6 micromoles d'alcool vératrylique en aldéhyde vératrique;

(b) a un poids moléculaire d'environ 38 kilo-daltons;

(c) contient un seul reste protohème IX;

(d) est glycosylée;

(e) contient les proportions molaires suivantes en amino-acides basées sur une mole d'isoleucine:

| asp/asn | 1,4 | thr | 2,2 | val | 1,6 |
|---------|-----|-----|-----|-----|-----|
| glu/gln | 6,0 | arg | 1,1 | phe | 1,1 |
| ser | 4,3 | ala | 7,3 | ile | 1,0 |
| his | 4,4 | tyr | 0,2 | leu | 1,5 |
| gly | 6,5 | met | non déterminé | lys | 0,5 |

(f) réagit dans un procédé immunoblot avec des anticorps polyclonaux dirigés contre les enzymes ligninolytiques (A) et (B) de la revendication 1;

(g) est éluée sur colonne FPLC à une molarité d'acétate de sodium d'environ 0,16; et

(h) a les activités suivantes sur des substrats modèles de la lignine

(1) clivage oxydant de $C_\alpha$-$C_\beta$;

(2) hydroxylation des groupes méthylène benzyliques;

(3) oxydation des alcools benzylique en aldéhydes;

(4) oxydation des phénols; et

(5) clivage oxydant des groupes méthoxy.

3. L'enzyme ligninolytique qui est substantiellement exempte de protéases et a les caractéristiques suivantes :

(a) 1 mg catalyse en une minute à environ 24°C l'oxydation d'environ 5,1 micromoles d'alcool vératrylique en aldéhyde vératrique;

(b) a un poids moléculaire d'environ 42 kilo-daltons;

(c) contient un seul reste protohème IX;

(d) est glycosylée;

(e) contient les proportions molaires suivantes en amino-acides basées sur une mole de tyrosine :

| asp/asn | 5,4 | thr | non déterminé | val | 7,4 |
|---------|------|-----|---------------|-----|-----|
| glu/gln | 16,8 | arg | 2,9 | phe | 7,0 |
| ser | 14,0 | ala | 14,4 | ile | 4,1 |
| his | 7,3 | tyr | 1,0 | leu | 6,5 |
| gly | 24,0 | met | 1,2 | lys | 2,5 |

(f) réagit dans un procédé immunoblot avec des anticorps polyclonaux dirigés contre les enzymes ligninolytiques (A) et (B) de la revendication 1;

(g) est éluée sur colonne FPLC à une molarité d'acétate de sodium d'environ 0,34; et

(h) a les activités suivantes sur des substrats modèles de la lignine;

(1) clivage oxyde de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant des groupes méthoxy.

4. L'enzyme ligninolytique qui est substantiellement exempte de protéases et a les caractéristiques suivantes :

(a) 1 mg catalyse en une minute à environ 24°C l'oxydation d'environ 9,7 micromoles d'alcool vératrylique en aldéhyde vératrique;

(b) a un poids moléculaire d'environ 42 kilo-daltons;

(c) contient un seul reste protohème IX;

(d) est glycosylée;

(e) réagit dans un procédé immunoblot avec des anticorps polyclonaux dirigés contre les enzymes ligninolytiques (A) et (B) de la revendication 1;

(f) est éluée sur colonne FPLC à une molarité d'acétate de sodium d'environ 0,40; et

(g) a les activités suivantes sur les substrats modèles de la lignine;

(1) clivage oxydant de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant des groupes méthoxy.

5. L'enzyme ligninolytique qui est substantiellement exempte de protéases et a les caractéristiques suivantes :

15

(a) 1 mg catalyse en une minute à environ 24°C l'oxydation d'environ 9,4 micromoles d'alcool vératrylique en aldéhyde vératrique;

(b) a un poids moléculaire d'environ 43 kilo-daltons;

(c) contient un seul reste protohème IX;

(d) est glycosylée;

(e) contient les proportions suivantes en amino-acides basées sur une mole de tyrosine :

| asp/asn | 5,0 | thr | non déterminé | val | 6,5 |
|---------|------|-----|---------------|-----|-----|
| glu/gln | 19,9 | arg | 4,8 | phe | 3,3 |
| ser | 22,3 | ala | 13,8 | ile | 3,6 |
| his | 15,9 | tyr | 1,0 | leu | 6,0 |
| gly | 44,7 | met | non déterminé | lys | 2,3 |

(f) réagit dans un procédé immunoblot avec des anticorps polyclonaux dirigés contre les enzymes ligninolytiques (A) et (B) de la revendication 1;

(g) est éluée sur colonne FPLC à une molarité d'acétate de sodium d'environ 0,58; et

(h) a les activités suivantes sur des substrats modèles de la lignine;

    (1) clivage oxydant de $C_\alpha$-$C_\beta$;

    (2) hydroxylation des groupes méthylène benzyliques;

    (3) oxydation des alcools benzyliques en aldéhydes;

    (4) oxydation des phénols; et

    (5) clivage oxydant des groupes méthoxy.

6. Une culture biologiquement pure de mutant de Phanerochaete chrysosporium, désignée mutant SC26, et ayant le numéro de dépôt de culture NRRL 15978, lequel mutant, par croissance dans un milieu approprié, élabore des enzymes ligninolytiques selon les revendications précédentes.

7. Un procédé de traitement d'une matière susceptible d'être dégradée par des enzymes ligninolytiques, caractérisé en ce que ladite matière est traitée par une enzyme ligninolytique selon l'une quelconque des revendications 1 à 5.

8. Un procédé selon la revendication 7, dans lequel ladite matière comprend de la lignine.

9. Un procédé selon la revendication 7, dans lequel ladite matière comprend n'importe quel substrat modèle de la lignine : alcool vératrylique ; éther 1-(3',4'-diméthoxyphénylique) et $\beta$-guaiacylique du glycérol; phénol; et un benzène méthoxylé, par exemple le 1,4-diméthoxybenzène.

10. Un procédé selon la revendication 8, dans lequel ladite matière est de la pâte kraft ou de la pâte thermomécanique.

**Revendications pour l'Etat contractant suivant : AT**

1. Un procédé de préparation de l'enzyme ligninolytique (A) qui est substantiellement exempte de protéases et a les caractéristiques suivantes :

    (a) 1 mg catalyse en une minute à environ 24°C l'oxydation d'environ 12,4 micromoles d'alcool vératrylique en aldéhyde vératrique;

    (b) a un poids moléculaire d'environ 42 kilo-daltons;

    (c) contient un seul reste protohème IX;

    (d) est glycosylée;

    (e) contient les proportions molaires suivantes en amino-acides basées sur une mole de lysine :

| asp/asn | 3,0 | thr | 4,9 | val | 4,2 |
|---------|-----|-----|-----|-----|-----|
| glu/gln | 8,0 | arg | 1,3 | phe | 3,2 |
| ser | 6,8 | ala | 6,7 | ile | 2,4 |
| his | 3,2 | tyr | 0,2 | leu | 3,3 |
| gly | 8,3 | met | 0,14 | lys | 1,0 |

(f) est éluée sur colonne FPLC à une molarité d'acétate de sodium d'environ 0,43; et

(g) a les activités suivantes sur des substrats modèles de la lignine;

(1) clivage oxydant de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant les groupes méthoxy; ou

de l'enzyme ligninolytique (B) qui est substantiellement exempte de protéases et a les caractéristiques suivantes:

(a) 1 mg catalyse en une minute à environ 24°C l'oxydation d'environ 17,1 micromoles d'alcool vératrylique en aldéhyde vératrique;

(b) a un poids moléculaire d'environ 38 kilo-daltons;

(c) contient un seul reste protohème IX;

(d) est glycosylée;

(d) contient les proportions molaires suivantes en amino-acides basées sur une mole de lysine :

| asp/asn | 2,0 | thr | 3,5 | val | 2,6 |
|---------|-----|-----|-----|-----|-----|
| glu/gln | 7,7 | arg | 1,2 | phe | 3,0 |
| ser | 4,1 | ala | 7,9 | ile | 2,2 |
| his | 3,2 | tyr | non déterminé | leu | 2,6 |
| gly | 5,7 | met | non déterminé | lys | 1,0 |

(f) est éluée sur colonne FPLC à une molarité d'acétate de sodium d'environ 0,18; et

(g) a les activités suivantes sur des substrats modèles de la lignine;

(1) clivage oxydant de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant des groupes méthoxy;

ladite enzyme ligninolytique (A) ayant la caractéristique de réagir dans un procédé immunoblot avec des anticorps polyclonaux dirigés contre les enzymes ligninolytiques (A) et (B); et ladite enzyme ligninolytique (B) ayant la caractéristique de réagir dans un procédé immunoblot avec des anticorps polyclonaux dirigés contre l'enzyme ligninolytique (A), lequel procédé comprend le traitement du fluide extracellulaire d'une culture de la souche SC26 de Phanerochaete chrysosporium (NPRL 15978), et la séparation de l'enzyme A ou de l'enzyme B par chromatographie échangeuse d'anions.

2. Un procédé de préparation de l'enzyme ligninolytique qui est substantiellement exempte de protéases et a les caractéristiques suivantes :

(a) 1 mg catalyse en une minute à environ 24°C l'oxydation d'environ 2,6 micromoles d'alcool vératrylique en aldéhyde vératrique;

(b) a un poids moléculaire d'environ 38 kilo-daltons;

(c) contient un seul reste protohème IX;

(d) est glycosylée;

(e) contient les proportions molaires suivantes en amino-acides basées sur une mole d'isoleucine:

EP 0 229 171 B1

| asp/asn | 1,4 | thr | 2,2 | val | 1,6 |
|---------|-----|-----|-----|-----|-----|
| glu/gln | 6,0 | arg | 1,1 | phe | 1,1 |
| ser | 4,3 | ala | 7,3 | ile | 1,0 |
| his | 4,4 | tyr | 0,2 | leu | 1,5 |
| gly | 6,5 | met | non déterminé | lys | 0,5 |

(f) réagit dans un procédé immunoblot avec des anticorps polyclonaux dirigés contre les enzymes ligninolytiques (A) et (B) de la revendication 1;

(g) est éluée sur colonne FPLC à une molarité d'acétate de sodium d'environ 0,16; et

(h) a les activités suivantes sur des substrats modèles de la lignine;

(1) clivage oxydant de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant des groupes méthoxy,

lequel procédé comprend le traitement du fluide extracellulaire d'une culture de la souche SC26 de Phanerochaete chrysosporium (NRRL 15978), et la séparation de ladite enzyme par chromatoraphie échangeuse d'anions.

3. Un procédé de préparation de l'enzyme ligninolytique qui est substantiellement exempte de protéases et a les caractéristiques suivantes :

(a) 1 mg catalyse en une minute à environ 24°C l'oxydation d'environ 5,1 micromoles d'alcool vératrylique en aldéhyde vératrique;

(b) a un poids moléculaire d'environ 42 kilo-daltons;

(c) contient un seul reste protohème IX;

(d) est glycosylée;

(e) contient les proportions molaires suivantes en amino-acides basées sur une mole de tyrosine:

| asp/asn | 5,4 | thr | non déterminé | val | 7,4 |
|---------|-----|-----|---------------|-----|-----|
| glu/gln | 16,8 | arg | 2,9 | phe | 7,0 |
| ser | 14,0 | ala | 14,4 | ile | 4,1 |
| his | 7,3 | tyr | 1,0 | leu | 6,5 |
| gly | 24,0 | met | 1,2 | lys | 2,5 |

(f) réagit dans un procédé immunoblot avec des anticorps polyclonaux dirigés contre les enzymes ligninolytiques (A) et (B) de la revendication 1;

(g) est éluée sur colonne FPLC à une molarité d'acétate de sodium d'environ 0,34; et

(h) a les activités suivantes sur des substrats modèles de la lignine;

(1) clivage oxydant de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant des groupes méthoxy,

lequel procédé comprend le traitement du fluide extracellulaire d'une culture de la souche SC26 de Phanerochaete chrysosporium (NRPL 15978), et la séparation de ladite enzyme par chromatographie échangeuse d'anions.

4. Un procédé de préparation de l'enzyme ligninolytique qui est substantiellement exempte de protéases et a les caractéristiques suivantes :

(a) 1 mg catalyse en une minute à environ 24°C l'oxydation d'environ 9,7 micromoles d'alcool vératrylique en aldéhyde vératrique;

(b) a un poids moléculaire d'environ 42 kilo-daltons;

(c) contient un seul reste protohème IX ;

18

(d) est glycosylée;

(e) réagit dans un procédé immunoblot avec des anticorps polyclonaux dirigés contre les enzymes ligninolytiques (A) et (B) de la revendication 1;

(f) est éluée sur colonne FPLC à une molarité d'acétate de sodium d'environ 0,40; et

(g) a les activités suivantes sur des substrats modèles de la lignine :

(1) clivage oxydant de $C_\alpha$-$C_\beta$;

(2) hydroxylation des groupes méthylène benzyliques;

(3) oxydation des alcools benzyliques en aldéhydes;

(4) oxydation des phénols; et

(5) clivage oxydant des groupes méthoxy,

lequel procédé comprend le traitement du fluide extracellulaire d'une culture de la souche SC26 de Phanerochaete chrysosporium (NRRL 15978), et la séparation de ladite enzyme par chromatographie échangeuse d'anions.

5. Un procédé de préparation de l'enzyme ligninolytique qui est substantiellement exempte de protéases et a les caractéristiques suivantes :

(a) 1 mg catalyse en une minute à environ 24°C l'oxydation d'environ 9,4 micromoles d'alcool vératrylique en aldéhyde vératrique;

(b) a un poids moléculaire d'environ 43 kilo-daltons;

(c) contient un seul reste prothème IX;

(d) est glycosylée;

(e) contient les proportions suivantes en amino-acides basées sur une mole de tyrosine :

```
asp/asn   5,0      thr   non déterminé      val   6,5

glu/gln  19,9      arg   4,8                phe   3,3

ser      22,3      ala  13,8               ile   3,6

his      15,9      tyr   1,0               leu   6,0

gly      44,7      met   non déterminé      lys   2,3
```

(f) réagit dans un procédé immunoblot avec des anticorps polyclonaux dirigés contre les enzymes ligninolytiques (A) et (B) de la revendication 1;

(g) est éluée sur colonne FPLC à une molarité d'acétate de sodium d'environ 0,58; et

(h) a les activités suivantes sur des substrats modèles de la lignine :

(1) clivage oxydant de $C_\alpha$-$C_\beta$;

(2) hydroxydation des groupes méthylène benzyliques;

(3) oxydation des alcools benzyliques en aldéhydes;

(4) oxydation des phénols; et

(5) clivage oxydant des groupes méthoxy,

lequel procédé comprend le traitement du fluide extracellulaire d'une culture de la souche SC26 de Phanerochaete chrysosporium (NRRL 15978), et la séparation de ladite enzyme par chromatographie échangeuse d'anions.

6. Un procédé de préparation d'une culture biologiquement pure d'un mutant de Phanerochaete chrysosporium , désigné mutant SC26, ayant le numéro de dépôt de culture NPRL 15978, lequel procédé comprend l'inoculation d'un milieu approprié avec ladite culture de mutant et la croissance de la culture sous des conditions appropriées pour permettre à ladite culture d'élaborer des enzymes ligninolytiques selon les revendications précédentes.

7. Un procédé de traitement d'une matière susceptible d'être dégradée par des enzymes ligninolytiques, caractérisé en ce que ladite matière est traitée par une enzyme ligninolytique selon l'une quelconque des revendications 1 à 5.

8. Un procédé selon la revendication 7, dans lequel ladite matière comprend de la lignine.

**9.** Un procédé selon la revendication 7, dans lequel ladite matière comprend n'importe quel substrat modèle de la lignine : alcool vératrylique ; éther 1-(3',4'-diméthoxyphénylique) et *β*-guaiacylique du glycérol; phénol; et un benzène méthoxylé, par exemple le 1,4-diméthoxybenzène.

**10.** Un procédé selon la revendication 8, dans lequel ladite matière est de la pâte kraft ou de la pâte thermomécanique.

**Patentansprüche**
**Patentansprüche für folgende Vertragsstaaten : BE, CH, DE, FR, GB, IT, LI, LU, NL, SE**

**1.** Das Lignin lösende Enzym (A) welches im wesentlichen frei ist von Proteasen und folgende Charakteristika aufweist:

a) 1 mg katalysiert in einer Minute bei etwa 24°C die Oxidation von etwa 12.4 Mikromol Veratrylalkohol zu Veratrylaldehyd;

b) hat ein Molekulargewicht von etwa 42 Kilo-Dalton;

c) enthält eine einzelne Häm IX Komponente;

d) ist glykosiert;

e) enthält die folgenden molaren Anteile von Aminosäuren bezogen auf 1 Mol Lysin:

| Asp/Asn | 3.0 | Thr | 4.9 | Val | 4.2 |
|---------|-----|-----|------|-----|-----|
| Glu/Gln | 8.0 | Arg | 1.3 | Phe | 3.2 |
| Ser | 6.8 | Ala | 6.7 | Ile | 2.4 |
| His | 3.2 | Tyr | 0.2 | Leu | 3.3 |
| Gly | 8.3 | Met | 0.14 | Lys | 1.0 |

f) eluiert von einer FPLC Säule bei einer Natriumacetat Molarität von etwa 0.43; und

g) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen; oder

das Lignin lösende Enzym (B) welches im wesentlichen frei ist von Proteasen und folgende Charakteristika aufweist:

a) 1 mg katalysiert in einer Minute bei etwa 24°C die Oxidation von etwa 17.1 Mikromol Veratrylalkohol zu Veratrylaldehyd;

b) hat ein Molekulargewicht von etwa 38 Kilo-Dalton;

c) enthält eine einzelne Häm IX Komponente;

d) ist glykosiert;

e) enthält die folgenden molaren Anteile von Aminosäuren bezogen auf 1 Mol Lysin:

| Asp/Asn | 2.0 | Thr | 3.5 | Val | 2.6 |
|---------|-----|-----|----------------|-----|-----|
| Glu/Gln | 7.7 | Arg | 1.2 | Phe | 3.0 |
| Ser | 4.1 | Ala | 7.9 | Ile | 2.2 |
| His | 3.2 | Tyr | nicht bestimmt | Leu | 2.6 |
| Gly | 5.7 | Met | nicht bestimmt | Lys | 1.0 |

f) eluiert von einer FPLC Säule bei einer Natriumacetat Molarität von etwa 0.18; und

g) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen;

dieses Lignin lösende Enzym (A) ist dadurch charakterisiert, dass es in einem Immunblot-Verfahren mit polyklonalen Antikörpern reagiert die verarbeitet werden von den Lignin lösenden Enyzmen (A) und (B) ausgelöst wurden; und das Lignin lösende Enzym (B) ist dadurch charakterisiert, dass es in einem Immunblot-Verfahren mit polyklonalen Antikörpern reagiert die vom Lignin lösenden Enzym (A) ausgelöst wurden.

2. Das Lignin lösende Enzym, welches im wesentlichen frei ist von Proteasen und folgende Charakteristika aufweist:

a) 1 mg katalysiert in einer Minute bei etwa 24°C die Oxidation von etwa 2.6 Mikromol Veratrylalkohol zu Veratrylaldehyd;

b) hat ein Molekulargewicht von etwa 38 Kilo-Dalton;

c) enthält eine einzelne Häm IX Komponente;

d) ist glykosiert;

e) enthält die folgenden molaren Anteile von Aminosäuren bezogen auf 1 Mol Isoleucin:

| Asp/Asn | 1.4 | Thr | 2.2 | Val | 1.6 |
|---------|-----|-----|-----|-----|-----|
| Glu/Gln | 6.0 | Arg | 1.1 | Phe | 1.1 |
| Ser | 4.3 | Ala | 7.3 | Ile | 1.0 |
| His | 4.4 | Tyr | 0.2 | Leu | 1.5 |
| Gly | 6.5 | Met | nicht bestimmt | Lys | 0.5 |

f) reagiert in einem Immunblot-Verfahren mit polyklonalen Antikörpern die von Lignin lösenden Enzymen (A) und (B) gemäss Anspruch 1 ausgelöst wurden;

g) eluiert von einer FPLC Säule bei einer Natriumacetat Molarität von etwa 0.16; und

h) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

3. Das Lignin lösende Enzym, welches im wesentlichen frei ist von Proteasen und folgende Charakteristika aufweist:

a) 1 mg katalysiert in einer Minute bei etwa 24°C die Oxidation von etwa 5.1 Mikromol Veratrylalkohol zu Veratrylaldehyd;

b) hat ein Molekulargewicht von etwa 42 Kilo-Dalton;

c) enthält eine einzelne Häm IX Komponente;

d) ist glykosiert;

e) enthält die folgenden molaren Anteile von Aminosäuren bezogen auf 1 Mol Tyrosin:

| Asp/Asn | 5.4 | Thr | nicht bestimmt | Val | 7.4 |
|---------|-----|-----|-----|-----|-----|
| Glu/Gln | 16.8 | Arg | 2.9 | Phe | 7.0 |
| Ser | 14.0 | Ala | 14.4 | Ile | 4.1 |
| His | 7.3 | Tyr | 1.0 | Leu | 6.5 |
| Gly | 24.0 | Met | 1.2 | Lys | 2.5 |

f) reagiert in einem Immunblot-Verfahren mit polyklonalen Antikörpern die von Lignin lösenden Enzymen (A) und (B) gemäss Anspruch 1 ausgelöst wurden;

g) eluiert von einer FPLC Säule bei einer Natriumacetat Molarität von etwa 0.34; und

h) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

4. Das Lignin lösende Enzym, welches im wesentlichen frei ist von Proteasen und folgende Charakteristika aufweist:

a) 1 mg katalysiert in einer Minute bei etwa 24 ° C die Oxidation von etwa 9.7 Mikromol Veratrylalkohol zu Veratrylaldehyd;

b) hat ein Molekulargewicht von etwa 42 Kilo-Dalton; c) enthält eine einzelne Häm IX Komponente;

d) ist glykosiert;

e) reagiert in einem Immunblot-Verfahren mit polyklonalen Antikörpern die von Lignin lösenden Enzymen (A) und (B) gemäss Anspruch 1 ausgelöst wurden;

f) eluiert von einer FPLC Säule bei einer Natriumacetat Molarität von etwa 0.40; und

g) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

5. Das Lignin lösende Enzym, welches im wesentlichen frei ist von Proteasen und folgende Charakteristika aufweist:

a) 1 mg katalysiert in einer Minute bei etwa 24 ° C die Oxidation von etwa 9.4 Mikromol Veratrylalkohol zu Veratrylaldehyd;

b) hat ein Molekulargewicht von etwa 43 Kilo-Dalton;

c) enthält eine einzelne Häm IX Komponente;

d) ist glykosiert;

e) enthält die folgenden Anteile von Aminosäuren bezogen auf 1 Mol Tyrosin:

| Asp/Asn | 5.0 | Thr | nicht bestimmt | Val | 6.5 |
| Glu/Gln | 19.9 | Arg | 4.8 | Phe | 3.3 |
| Ser | 22.3 | Ala | 13.8 | Ile | 3.6 |
| His | 15.9 | Tyr | 1.0 | Leu | 6.0 |
| Gly | 44.7 | Met | nicht bestimmt | Lys | 2.3 |

f) reagiert in einem Immunblot-Verfahren mit polyklonalen Antikörpern die von Lignin lösenden Enzymen (A) und (B) gemäss Anspruch 1 ausgelöst wurden;

g) eluiert von einer FPLC Säule bei einer Natriumacetat Molarität von etwa 0.58; und

h) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

6. Eine biologisch reine mutante Kultur von Phanerochaete chrysosporium bezeichnet als Stamm SC 26 mit der Hinterlegungsnummer NRRL 15978 welcher Mutant nachdem er in einem geeigneten Medium gewachsen ist Lignin lösende Enzyme gemäss der vorausgehenden Ansprüche entwickelt.

7. Ein Verfahren zum Behandeln eines Materials das gegen Zersetzung empfindlich ist mit Lignin lösenden Enzymen, dadurch gekennzeichnet, dass dieses Material mit einem Lignin lösenden Enzym gemäss einem der Ansprüche 1 bis 5 behandelt wird.

8. Ein Verfahren gemäss Anspruch 7, dadurch gekennzeichnet, dass dieses Material Lignin enthält.

9. Ein Verfahren gemäss Anspruch 7, dadurch gekennzeichnet, dass dieses Material eines der folgenden Lignin Modell Substrate enthält: Veratrylalkohol, 1-(3',4'-dimethoxyphenyl)-Glyzerin-$\beta$-Guajakoläther; Phenol; sowie methoxyliertes Benzol wie z.B. 1,4-Dimethoxybenzol.

10. Ein Verfahren gemäss Anspruch 8, dadurch gekennzeichnet, dass dieses Material Kraftzellstoff oder thermomechanischer Holzstoff ist.

**Patentansprüche für folgenden Vertragsstaat: AT**

1. Ein Verfahren zur Herstellung eines Lignin lösenden Enzyms (A) welches im wesentlichen frei von Proteasen ist und folgende Charakteristika aufweist:

a) 1 mg katalysiert in einer Minute bei etwa 24°C die Oxidation von etwa 12.4 Mikromol Veratrylalkohol zu Veratrylaldehyd;

b) hat ein Molekulargewicht von etwa 42 Kilo-Dalton;

c) enthält eine einzelne Häm IX Komponente;

d) ist glykosiert;

e) enthält die folgenden molaren Anteile von Aminosäuren bezogen auf 1 Mol Lysin:

| Asp/Asn | 3.0 | Thr | 4.9 | Val | 4.2 |
|---------|-----|-----|------|-----|-----|
| Glu/Gln | 8.0 | Arg | 1.3 | Phe | 3.2 |
| Ser | 6.8 | Ala | 6.7 | Ile | 2.4 |
| His | 3.2 | Tyr | 0.2 | Leu | 3.3 |
| Gly | 8.3 | Met | 0.14 | Lys | 1.0 |

f) eluiert von einer FPLC Säule bei einer Natriumacetat Molarität von etwa 0.43; und

g) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen; oder

das Lignin lösende Enzym (B) welches im wesentlichen frei ist von Proteasen und folgende Charakteristika aufweist:

a) 1 mg katalysiert in einer Minute bei etwa 24°C die Oxidation von etwa 17.1 Mikromol Veratrylalkohol zu Veratrylaldehyd;

b) hat ein Molekulargewicht von etwa 38 Kilo-Dalton;

c) enthält eine einzelne Häm IX Komponente;

d) ist glykosiert;

e) enthält die folgenden molaren Anteile von Aminosäuren bezogen auf 1 Mol Lysin:

| Asp/Asn | 2.0 | Thr | 3.5 | Val | 2.6 |
|---------|-----|-----|-----|-----|-----|
| Glu/Gln | 7.7 | Arg | 1.2 | Phe | 3.0 |
| Ser | 4.1 | Ala | 7.9 | Ile | 2.2 |
| His | 3.2 | Tyr | nicht bestimmt | Leu | 2.6 |
| Gly | 5.7 | Met | nicht bestimmt | Lys | 1.0 |

f) eluiert von einer FPLC Säule bei einer Natriumacetat Molarität von etwa 0.18; und

g) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen;

dieses Lignin lösende Enzym (A) ist dadurch charakterisiert, dass es in einem Immunblot-Verfahren mit polyklonalen Antikörpern reagiert die von den Lignin lösenden Enyzmen (A) und (B) ausgelöst wurden; und das Lignin lösende Enzym (B) ist dadurch charakterisiert, dass es in einem Immunblot-Verfahren mit polyklonalen Antikörpern reagiert die vom Lignin lösenden Enzym (A) ausgelöst wurden, welches Verfahren darin besteht die extrazellulare Flüssigkeit einer Kultur von Phanerochaete chrysosporium, Stamm SC 26 (NRRL 15978) aufzuarbeiten und das Enzym (A) oder (B) mittels Anionaustauschchromatographie abzutrennen.

**2.** Ein Verfahren zur Herstellung eines Lignin lösenden Enzyms welches im wesentlichen frei ist von Proteasen und folgende Charakteristika aufweist:

a) 1 mg katalysiert in einer Minute bei etwa 24°C die Oxidation von etwa 2.6 Mikromol Veratrylalkohol zu Veratrylaldehyd;

b) hat ein Molekulargewicht von etwa 38 Kilo-Dalton;

c) enthält eine einzelne Häm IX Komponente;

d) ist glykosiert;

e) enthält die folgenden molaren Anteile von Aminosäuren bezogen auf 1 Mol Isoleucin:

| Asp/Asn | 1.4 | Thr | 2.2 | Val | 1.6 |
|---------|-----|-----|----------------|-----|-----|
| Glu/Gln | 6.0 | Arg | 1.1 | Phe | 1.1 |
| Ser | 4.3 | Ala | 7.3 | Ile | 1.0 |
| His | 4.4 | Tyr | 0.2 | Leu | 1.5 |
| Gly | 6.5 | Met | nicht bestimmt | Lys | 0.5 |

f) reagiert in einem Immunblot-Verfahren mit polyklonalen Antikörpern die von Lignin lösenden Enzymen (A) und (B) gemäss Anspruch 1 ausgelöst wurden;

g) eluiert von einer FPLC Säule bei einer Natriumacetat Molarität von etwa 0.16; und

h) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen,

welches Verfahren darin besteht die extrazellulare Flüssigkeit einer Kultur von Phanerochaete chrysosporium, Stamm SC 26 (NRRL 15978) aufzuarbeiten und das Enzym mittels Anionaustauschchromatographie abzutrennen.

**3.** Ein Verfahren zur Herstellung des Lignin lösenden Enzyms, welches im wesentlichen frei ist von Proteasen und folgende Charakteristika aufweist:

a) 1 mg katalysiert in einer Minute bei etwa 24°C die Oxidation von etwa 5.1 Mikromol Veratrylalkohol zu Veratrylaldehyd;

b) hat ein Molekulargewicht von etwa 42 Kilo-Dalton;

c) enthält eine einzelne Häm IX Komponente;

d) ist glykosiert;

e) enthält die folgenden molaren Anteile von Aminosäuren bezogen auf 1 Mol Tyrosin:

| Asp/Asn | 5.4 | Thr | nicht bestimmt | Val | 7.4 |
|---------|------|-----|----------------|-----|-----|
| Glu/Gln | 16.8 | Arg | 2.9 | Phe | 7.0 |
| Ser | 14.0 | Ala | 14.4 | Ile | 4.1 |
| His | 7.3 | Tyr | 1.0 | Leu | 6.5 |
| Gly | 24.0 | Met | 1.2 | Lys | 2.5 |

f) reagiertin einem Immunblot-Verfahren mit polyklonalen Antikörpern die von Lignin lösenden Enzymen (A) und (B) gemäss Anspruch 1 asugelöst wurden;

g) eluiert von einer FPLC Säule bei einer Natriumacetat Molarität von etwa 0.34; und

h) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

welches Verfahren darin besteht die extrazellulare Flüssigkeit einer Kultur von Phanerochaete chrysosporium, Stamm SC26 (NRRL 15978) aufzuarbeiten und das Enzym mittels Anionaustauschchromatographie abzutrennen.

4. Ein Verfahren zur Herstellung des Lignin lösenden Enzyms, welches im wesentlichen frei ist von Proteasen und folgende Charakteristika aufweist:

a) 1 mg katalysiert in einer Minute bei etwa 24°C die Oxidation von etwa 9.7 Mikromol Veratrylalkohol zu Veratrylaldehyd;

b) hat ein Molekulargewicht von etwa 42 Kilo-Dalton;

c) enthält eine einzelne Häm IX Komponente;

d) ist glykosiert;

e) reagiert in einem Immunblot-Verfahren mit polyklonalen Antikörpern die von Lignin lösenden Enzymen (A) und (B) gemäss Anspruch 1 ausgelöst;

f) eluiert von einer FPLC Säule bei einer Natriumacetat Molarität von etwa 0.40; und

g) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

welches Verfahren darin besteht die extrazellulare Flüssigkeit einer Kultur von Phanerochaete chrysosporium, Stamm SC 26 (NRRL 15978) aufzuarbeiten und das Enzym mittels Anionaustauschchromatographie abzutrennen.

5. Ein Verfahren zur Herstellung des Lignin lösenden Enzyms, welches im wesentlichen frei ist von Proteasen und folgende Charakteristika aufweist:

a) 1 mg katalysiert in einer Minute bei etwa 24°C die Oxidation von etwa 9.4 Mikromol Veratrylalkohol zu Veratrylaldehyd;

b) hat ein Molekulargewicht von etwa 43 Kilo-Dalton;

c) enthält eine einzelne Häm IX Komponente;

d) ist glykosiert;

e) enthält die folgenden Anteile von Aminosäuren bezogen auf 1 Mol Tyrosin:

| | | | | | |
|---|---|---|---|---|---|
| Asp/Asn | 5.0 | Thr | nicht bestimmt | Val | 6.5 |
| Glu/Gln | 19.9 | Arg | 4.8 | Phe | 3.3 |
| Ser | 22.3 | Ala | 13.8 | Ile | 3.6 |
| His | 15.9 | Tyr | 1.0 | Leu | 6.0 |
| Gly | 44.7 | Met | nicht bestimmt | Lys | 2.3 |

f) reagiert in einem Immunblot-Verfahren mit polyklonalen Antikörpern die von Lignin lösenden Enzymen (A) und (B) gemäss Anspruch 1 ausgelöst wurden;

g) eluiert von einer FPLC Säule bei einer Natriumacetat Molarität von etwa 0.58; und

h) hat die folgenden Wirksamkeiten auf Lignin Modell Substraten:

(1) Oxydative Spaltung von $C_\alpha$-$C_\beta$

(2) Hydroxilierung von benzylischen Methylengruppen;

(3) Oxidation von Benzylalkoholen zu Aldehyden;

(4) Phenoloxidation; und

(5) Oxidative Spaltung von Methoxylgruppen.

welches Verfahren darin besteht die extrazellulare Flüssigkeit einer Kultur von Phanerochaete chrysosporium, Stamm SC 26 (NRRL 15978) aufzuarbeiten und das Enzym mittels Anionaustauschchromatographie abzutrennen.

6. Ein Verfahren zur Herstellung einer biologisch reinen mutanten Kultur von Phanerochaete chrysosporium bezeichnet als Stamm SC 26 mit der Hinterlegungsnummer NRRL 15978, dadurch gekennzeichnet, dass man ein geeignetes Medium mit dieser mutanten Kultur impft, und die Kultur unter Bedingungen wachsen lässt die geeignet sind Lignin lösende Enzyme gemäss der vorausgehenden Ansprüche zu entwickeln.

7. Ein Verfahren zum Behandeln eines Materials das empfindlich gegen Zersetzung mit Lignin lösenden Enzymen ist, dadurch gekennzeichnet, dass dieses Material mit Lignin lösenden Enzymen gemäss einem der Ansprüche 1 bis 5 behandelt wird.

8. Ein Verfahren gemäss Anspruch 7, dadurch gekennzeichnet, dass dieses Material Lignin enthält.

9. Ein Verfahren gemäss Anspruch 7, dadurch gekennzeichnet, dass dieses Material eines der folgenden Lignin Modell Substrate enthält; Veratrylalkohol 1-(3',4'-dimethoxyphenyl)-Glyzerin-$\beta$-Guajakoläther; Phenol; sowie methoxyliertes Benzol, wie z.B. 1,4-Dimethoxybenzol.

10. Ein Verfahren gemäss Anspruch 8, dadurch gekennzeichnet, dass dieses Material Kraftzellstoff oder thermomechanischer Holzstoff ist.